# EUROPEAN PATENT SPECIFICATION

(11) **EP 1 853 331 B1**
(45) Date of publication and mention of the grant of the patent: **04.05.2011**
(21) Application number: 06736716.9
(22) Date of filing: 02.03.2006
(51) Int. Cl.: A61L 29/08, A61L 29/12, A61L 29/06, A61M 25/00, A61M 25/06

(54) **INTRODUCER SHEATH**
EINFÜHRSCHLEUSE
GAINE D'INTRODUCTION

(30) Priority: 02.03.2005 US 657777 P
(43) Date of publication of application: 14.11.2007
(60) Divisional application: 10176021.3
(73) Proprietor: Cook, Inc., Bloomington, IN 47404 (US)
(72) Inventor: LENTZ, D., Bloomington, IN 47401 (US)
(74) Representative: Garratt, Peter Douglas
(86) International application number: PCT/US2006/007444
(87) International publication number: WO 2006/094135

(56) References cited:
- EP-A2- 0 709 108
- WO-A-01/07231
- WO-A-2004/096338
- WO-A2-2004/002562
- WO-A2-2004/060464
- WO-A2-2006/028522
- US-A- 5 324 262
- US-A- 5 380 304
- US-A- 5 997 508
- US-A1- 2003 212 373
- US-B1- 6 596 818
- US-B1- 6 808 509

## Description

### BACKGROUND

Technical Field. This invention relates to the field of medical devices and, more particularly, to sheaths and catheters for use in introducing interventional devices and/or medicaments into the vascular system of a patient.

Background Information. Introducer sheaths are widely used for delivering interventional devices, such as a stent or a stent graft, or for delivering fluid medicaments, to a deployment site within the vasculature of a patient. However, sheaths used to deliver such devices and medicaments have a tendency to kink, particularly when it is necessary for the catheter to traverse tortuous pathways in the vasculature. Kinking reduces the effective inner diameter of the sheath, which often renders it unusable. The tendency to kink is increased when the sheath is used to introduce an interventional device into one of the many smaller vessels that branch off from major vessels. In this event, the sheath may have insufficient flexibility at the very point where flexibility is required in order to enable proper positioning of the interventional device.

In order to traverse the narrow confines of the vascular system, introducer sheaths are generally formed of thin-wall construction. Increasing the thickness of the sheath wall can minimally improve the level of kink resistance. Any such increase, however, is inherently undesirable since it necessitates the use of a larger entry opening than would otherwise be required, and limits the potential use of the device.

One introducer sheath with improved kink resistance is disclosed in U.S. Pat. No. 5,380,304 to Parker. The introducer sheath described in the '304 patent comprises an inner liner formed of a lubricious fluoropolymer, such as polytetrafluoroethylene (PTFE). A coil is compression-fitted around the inner PTFE liner. An outer jacket is formed of a heat-formable polyamide material, such as nylon. The nylon heat formable material is heat shrunk onto the PTFE outer surface by enveloping it in a heat shrink tube of fluorinated ethylene propylene (FEP), and heating the entire assembly until the nylon melts. As the nylon melts, it flows between the spacings of the coils and bonds to the outer diameter of the PTFE layer. The heat shrink tube is then skived from the assembly and discarded. The compression-fitted coil of this device reinforces the wall to provide an extremely kink-resistant and thin-walled introducer sheath.

The introducer sheath described in the '304 patent has proven to be particularly effective in delivering medical devices and medicaments to a patient's vasculature without kinking. However, the use of the heat shrink layer introduces certain inefficiencies into the sheath formation process. For example, the necessity to discard the heat shrink layer after use results in the waste of a component material. In addition, the necessity to skive the heat shrink layer introduces a source of inefficiency, resulting from the introduction of an additional step into the process. Furthermore, the skiving step must be performed with extreme precision to insure that the underlying nylon tubing layer is not inadvertently cut. If this occurs, the entire sheath must be discarded, thereby adding another layer of inefficiency to the overall manufacturing process. Still further, the use of conventional heat shrink tube materials necessitates the use of a higher melting temperature than would otherwise be required if lower melting materials could be utilized.

Another problem with existing introducer catheters and sheaths is that they sometimes lack sufficient stiffness when they are used to introduce certain interventional devices during an emergency procedure. Since most such sheaths have very small outer diameters, they are particularly prone to being bent and kinked under the time constraints that may arise during an emergency situation. If kinking occurs during such situations, the sheath becomes unusable and a new sheath must be introduced at the same or another access site. During emergency procedures, time is normally of the essence, and the necessity to repeat certain actions taken on the part of the medical team is inherently undesirable. In addition, the necessity to discard an unusable sheath and replace it with a new one adds unnecessary expense to the procedure.

It is desired to provide an introducer sheath that may be manufactured in an efficient manner, and that does not result in the waste of component materials. In addition, it is desired to provide an introducer sheath that has sufficient stiffness to permit it to be introduced into the vascular system to perform an interventional procedure, and yet has sufficient flexibility and kink resistance to permit it to be directed to one or more small branch vessels.

### BRIEF SUMMARY

The shortcomings of the prior art are addressed by the present invention. In one form thereof, the invention comprises an introducer sheath having an inner liner and a reinforcing member disposed radially outwardly of the inner liner. An intermediate layer is positioned longitudinally over the reinforcing member and inner liner, and is connected to an outer circumferential surface of the inner liner between spacings of the reinforcing member. An outer layer is positioned longitudinally over the intermediate layer. The outer layer comprises a polymer that is at least partially cross-linked, wherein the outer layer is bonded to the outer surface of the intermediate layer. Preferably, the outer layer comprises a block copolymer, such as polyether block amide, having alternate segments of hard and soft blocks, and the intermediate layer comprises a thermoplastic polymer, such as nylon.

In another form thereof, the present invention comprises an introducer sheath comprising a reinforced layer having a passageway extending therethrough, and an outer layer bonded to the outer surface of the reinforced layer. The outer layer comprises a cross-linked block copolymer having alternate segments of hard and soft blocks. Non-cross-linked segments of the block copolymer are bonded with active functional groups of the reinforced layer.

In still another form thereof, the present invention comprises a method for forming an introducer sheath. Initially, an assembly is provided comprising an inner liner, a reinforcement disposed over the inner liner, and a polymeric intermediate layer disposed over the inner liner. The assembly is positioned within a heat shrinkable material comprising a polymeric material capable of bonding with the polymeric intermediate layer. The heat shrinkable material and the assembly positioned therein are heated to a temperature sufficient to shrink the heat shrinkable material, and to melt the polymeric intermediate layer in a manner such that an outer surface of the melted polymeric intermediate material bonds to the heat shrinkable material, and an inner surface of the melted polymeric intermediate layer bonds to the inner liner.

### BRIEF DESCRIPTION OF THE DRAWINGS

Fig. 1 is an illustrative embodiment of a flexible, kink-resistant introducer sheath of the present invention;

Fig. 2 is a longitudinal cross-sectional view of a portion of the wall of the introducer sheath of Fig. 1, taken along line 2-2;

Fig. 3 is a transverse cross-sectional view of the introducer sheath of Fig. 1 taken along line 3-3, with the dilator removed;

Fig. 4 is a partial cross-sectional view of another embodiment of an introducer sheath, wherein the sheath has a coil reinforcement; and

Fig. 5 is a partial cross-sectional view of still another embodiment of an introducer sheath, wherein the sheath has a braid and a coil reinforcement.

### DETAILED DESCRIPTION OF THE DRAWINGS AND THE PRESENTLY PREFERRED EMBODIMENTS

For the purposes of promoting an understanding of the principles of the invention, reference will now be made to the embodiments illustrated in the drawings, and specific language will be used to describe the same. It should nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

In the following discussion, the terms "proximal" and "distal" will be used to describe the opposing axial ends of the inventive sheath, as well as the axial ends of various component features. The term "proximal" is used in its conventional sense to refer to the end of the apparatus (or component thereof) that is closest to the operator during use of the apparatus. The term "distal" is used in its conventional sense to refer to the end of the apparatus (or component thereof) that is initially inserted into the patient, or that is closest to the patient.

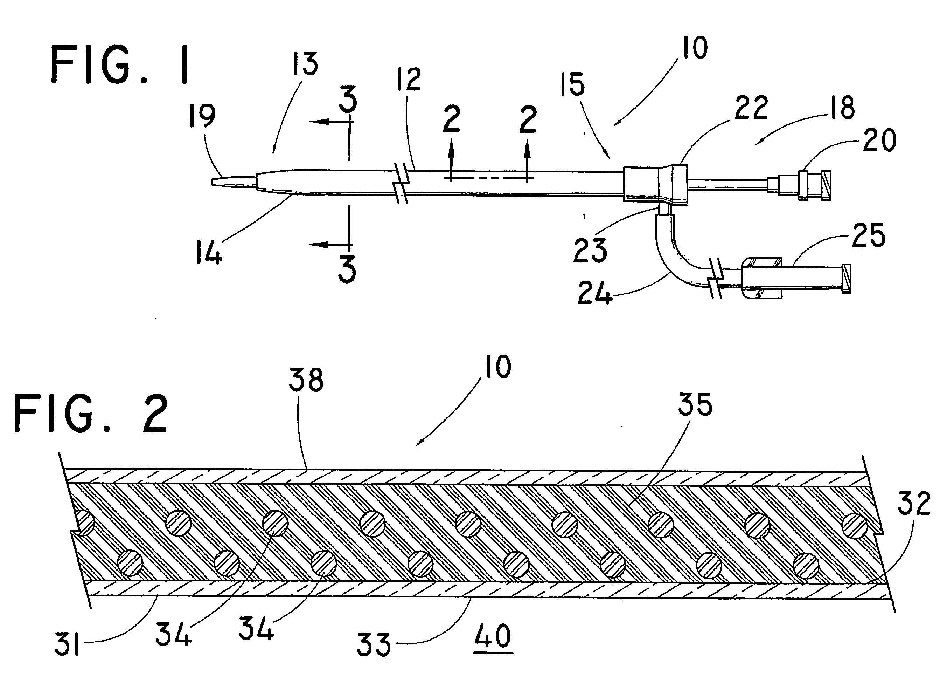

Fig. 1 shows an illustrative flexible introducer sheath 10 according to an embodiment of the present invention. Introducer sheath 10 includes an outer tube 12, having a distal portion 13 and a proximal portion 15. Preferably, distal portion 13 tapers to a tapered distal end 14.

In Fig. 1, sheath 10 is shown in combination with an optional dilator 18 that extends longitudinally through the passageway of the sheath, and a conventional connector hub 22 that is attached about the proximal end of the sheath. Dilator 18 includes a tapered distal end 19 for accessing and dilating a vascular access site, e.g., over a conventional wire guide (not shown). A male Luer lock connector hub 20 may be attached at the proximal end of the dilator for connection to syringes and other medical apparatus. Connector hub 22 may include a conventional silicone disk (not shown) for preventing the backflow of fluids therethrough. Connector hub 22 may also include a side arm 23, to which a polymeric tube 24 and a male Luer lock connector 25 may be connected for introducing and aspirating fluids therethrough in conventional fashion.

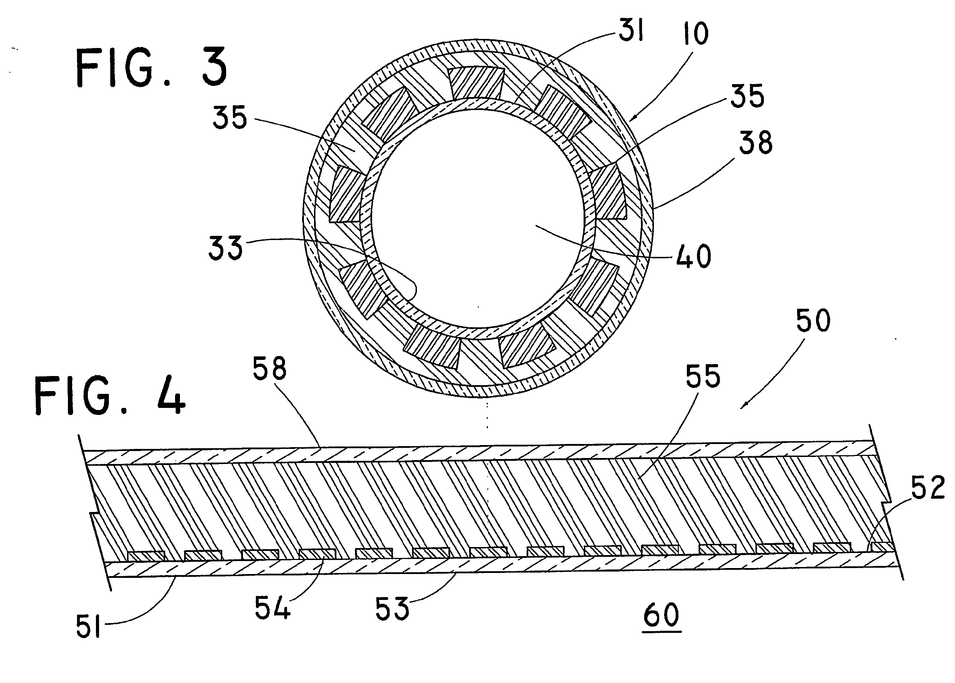

Fig. 2 is a longitudinal cross-sectional view of a portion of the wall of introducer sheath 10 according to an embodiment of the present invention. This figure illustrates the layered structure of the sheath. Fig. 3 is a transverse cross-sectional view of introducer sheath 10. Dilator 18 has been omitted from Figs. 2 and 3. As illustrated, sheath 10 comprises an inner liner 31, and a conventional reinforcing member, such as wire braid 34, fitted around inner liner 31. A polymeric intermediate layer 35 is mechanically connected to the outer surface 32 of inner liner 31 through the spaced filaments of the braid 34. An outer layer 38 is formed of a heat shrinkable material, such as a polyether block amide (PEBA). Outer layer 38 is bonded or otherwise fused onto polymeric intermediate layer 35.

Braid 34 comprises a plurality of crossed wires, and is preferably formed of medical grade metal or metal alloy. Non-limiting examples of such materials include stainless steel, and shape memory alloys such as nitinol, a nickel-titanium alloy. Braids are well-known reinforcements for medical devices. Those skilled in the art will appreciate that braid 34 may alternatively be formed of other medical grade materials known in the art for such use, such as polymers and composite materials. The braid may be formed with varied numbers, and pitches, of crossed wires, which number of wires and pitch may be varied within segments of a particular sheath, all in accordance with known techniques.

Fig. 4 illustrates a portion of another embodiment of an introducer sheath 50. The embodiment of Fig. 4 is similar to the embodiment illustrated in Fig. 2, except that the sheath includes a coil reinforcement 54, rather than a braid. In this case, coil 54 may be wrapped, wound or compression fitted around inner liner 51 in conventional fashion. Polymeric intermediate layer 55 is mechanically connected to roughened outer surface 52 of the inner liner through the coil windings. As with the braid described previously, coil 54 may comprise a metal or metal alloy, such as stainless steel, or alternatively may be formed from other conventional medical grade materials known in the art to be suitable for such use. Preferably, coil 54 is formed from flat wire. Outer layer 58 is formed of a heat shrinkable material, as before, and is bonded or otherwise fused onto polymeric intermediate layer 55.

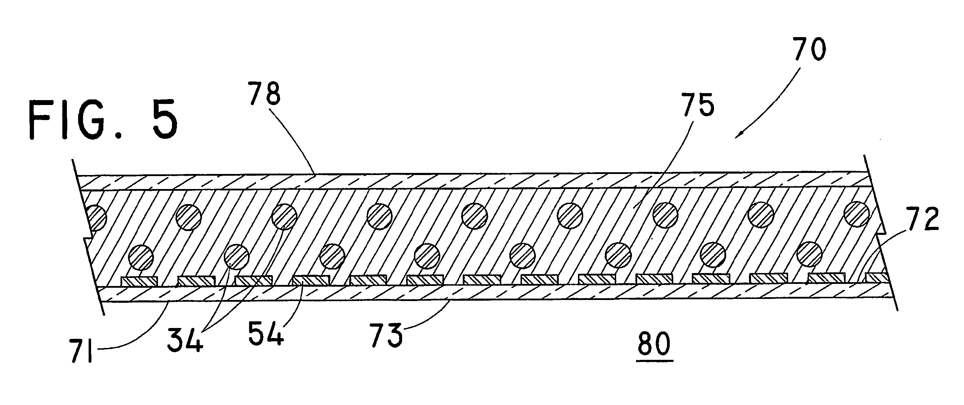

Fig. 5 illustrates a portion of yet another embodiment of an introducer sheath 70. This embodiment is similar to that of Figs. 2 and 4, except in this instance, sheath 70 incorporates both a coil 54 and a braid 34 reinforcement. Once again, polymeric intermediate layer 75 is mechanically connected to roughened outer surface 72 of the inner liner through the coil windings. Outer layer 78 is formed of a heat shrinkable material, as before, and is bonded or otherwise fused onto polymeric intermediate layer 75.

Typically, a coil reinforcement is utilized to prevent kinking or ovalizing of the sheath. A braid, on the other hand, is typically utilized to impart torque control to the device. Torque control is advantageous when maneuvering a lengthy sheath or catheter into distal anatomy. Utilizing both types of reinforcement provides both kink resistance and torque control in a single sheath. Although the embodiment of Fig. 5 includes the coil as the radially innermost of the two reinforcements, this need not be the case, and alternatively, the braid may be the innermost reinforcement. In all of the embodiments herein, the various reinforcements can extend the entire length of the sheath, or less than the entire length, all in accordance with known techniques.

In the embodiments of Figs. 2, 4 and 5, respectively, inner liner 31,51, 71 is typically formed of a lubricious material. Preferably, the lubricious material comprises a fluorocarbon, such as PTFE or FEP, or a polyimide. Lubricious inner liners for sheaths are well known in the medical arts, and those skilled in the art can readily select an appropriate liner for a particular use. The lubricous material provides a slippery inner surface 33, 53, 73 for easy insertion and withdrawal of a dilator or other interventional device through a passageway 40, 60, 80 extending longitudinally through the interior of respective sheath 10, 50, 70. Preferably, the radially outer surface 32, 52, 72 of inner liner 31, 51, 71 is roughened in any conventional manner, such as by chemical etching, to form a rough outer surface to facilitate bonding with polymeric intermediate layer 35, 55, 75. Inner liner 31, 51, 71 also preferably has a substantially uniform inner diameter that extends the entire length of passageway 40, 60, 80, to enable passage therethrough of the largest possible diameter interventional device. The wall of the inner liner will also preferably have sufficient structural integrity to prevent the wires of the reinforcement from protruding into inner passageway 40, 60, 80.

Polymeric intermediate layer 35, 55, 75 comprises a polymer that is positioned over and in contact with inner liner 31, 51, 71 and reinforcement 34, 54. The filaments of braid 34 in the embodiments of Figs. 2 and 5, and the wire turns of coil 54 in the embodiments of Figs. 4 and 5, are preferably spaced a sufficient distance from each other such that when the intermediate polymeric layer 35, 55, 75 is melted as described hereinafter, it flows between the braid filaments and/or the wire turns to bond to the roughened outer surface of the inner liner. In a preferred embodiment, polymeric intermediate layer 35, 55, 75 comprises a nylon (polyamide) or polyurethane, although other conventional medical grade materials known to be suitable for such use, such as PEBA and PET, may be substituted.

Outer layer 38, 58, 78 is formed of a heat shrinkable material that is capable of forming a secure bond with polymeric intermediate layer 35, 55, 75, The heat shrinkable material of the outer layer typically has a higher melting point than that of the material of the intermediate layer. In this manner, upon exposure to a controlled amount of heat, the intermediate layer melts and flows between the interstices of the braid and is fused to the roughened outer surface of the inner liner. The outer layer compresses the intermediate layer and the inner liner in well-known manner when heat shrink operations are utilized. Those skilled in the art will appreciate that the outer layer and the polymeric intermediate layer may be formed from a variety of medical grade materials suitable for such purposes, so long as the layers are capable of being fused or otherwise bonded or securely attached to each other upon the application of heat, as described. Unlike many prior art sheaths, the heat shrinkable outer layer is not skived off from the remainder of the sheath following the heat shrink, but rather, remains an integral part of the sheath.

When the intermediate layer comprises a nylon as described, a preferred outer layer material comprises a copolymer, and more preferably, a block copolymer such as polyether block amide (PEBA). Block copolymers comprise alternating segments formed of a harder, or more crystalline material, and a softer, or more amorphous, material. When the copolymer is PEBA, the harder material comprises a polyamide, such as nylon 12, and the amorphous segments comprise polyether. The hard and the amorphous segments are linked together by urethane groups in known fashion. As is well known in the art, by varying the ratio of the polyamide to polyether blocks, PEBA compositions of varying properties, such as melting point, dimensional stability, hardness, etc., may be created. Commercially available grades of PEBA typically have a Shore hardness between about 72D and 75A. Higher polyamide to polyether ratios will result in a higher Shore hardness (stiffer material), and lower polyamide to polyether ratios result in a lower Shore hardness (softer material).

Typically, the outer layer comprises a thermoplastic material that is subjected to at least partial cross-linking. When a thermoplastic material is cross-linked, chemical links are established between the molecular chains of the polymer, thereby resulting in a change of properties in the cross-linked material when compared to the non-cross-linked material. In general, when a thermoplastic material is cross-linked, the properties of the cross-linked material cause it to behave more in the nature of a thermoset material. Thus, the resulting thermoset-like material may have higher dimensional stability (hoop strength), higher tensile strength, higher stiffness and density, higher melting temperature, improved heat memory, improved chemical resistance, and improved physical strength, among other properties, when compared to the non-cross-linked thermoplastic. Similarly, some properties, such as elongation and the ability to flex, are generally lower in the cross-linked material when compared to the non-cross-linked material. Thus, for example, when the cross-linked material is a block copolymer such as PEBA, the properties of the resulting cross-linked material will generally differ from those of the original block copolymer in the manner described above.

Although cross-linking may be utilized to alter the properties of a polymeric material as described, it is not always necessary, or even desirable, to have a fully cross-linked material. For example, although the tensile strength of the polymer generally increases with increased cross-linking as described, there typically comes a point at which further cross-linking causes the polymer to become brittle. Once a polymer becomes brittle, it may be effectively unusable for a particular purpose. Similarly, the other desirable properties of a cross-linked material described above may reach a point of diminishing returns with increased cross-linking. Thus, it is often desirable to control the amount, or degree, of cross-linking of a particular block copolymer in order to optimize the desired properties of the cross-linked polymer. In some instances a trade-off must be made to arrive at a copolymer that is sufficiently cross-linked to be effective for its intended purpose, but not so highly cross-linked as to effectively negate the beneficial results of the cross-linking. Those skilled in the art are capable of determining such optimal conditions for a particular use upon routine experimentation.

Cross-linking procedures are well known in the arts. Typically, cross-linking is initiated by chemical means, or by irradiation. With chemical initiation, an initiating compound, such as a peroxide, is mixed into the matrix of the polymer. With irradiation, a material is exposed to high-energy radiation to initiate the formation of the molecular bonding. Both of these methods have been found to effectively promote molecular bonding within the material. In modem practice, irradiation is probably the more common mode for initiating the cross-linking reaction. Examples of procedures involving the cross-linking of polymeric compositions for use in medical applications are discussed, e.g., in U.S. Patent No. 6,663,646 and U.S. Patent No. 6,596,818,

In general, it is easier to control an irradiation than a chemical reaction, and an irradiation may be carried out at lower temperatures than most chemical cross-linking chemical reactions. Those skilled in the art are well aware of various chemical, and irradiation, means for cross-linking polymeric materials, and are able to select a suitable technique. Similarly, those skilled in the art recognize that routine experimentation may be required to determine the optimal conditions for a particular cross-linking operation, such as an optimal irradiation agent and an optimal time and manner of irradiation, for a particular desired outcome.

When the material to be cross-linked is PEBA, in which the polyamide blocks and the polyether blocks are separated by a urethane functional group as described, it is preferred to carry out the cross-linking reaction of the PEBA copolymer in a manner such that it is primarily the urethane groups that undergo cross-linking, and the polyamide groups are subject to only minimal, if any, cross-linking. Appropriate selection of the conditions of the cross-linking, such as the type of material to be cross-linked, the type of cross-linking that the material will undergo (i.e., chemical v. irradiation), the type of initiator utilized, and the time and conditions of the cross-link operation, among other well-known variables, enable the PEBA to be cross-linked in a manner to provide a satisfactory material for use in a particular operation. Suitably cross-linked materials may be obtained from commercial sources. One preferred cross-linked PEBA material suitable for use herein is available from Cobalt Polymers, of Cloverdale, California. This cross-linked material is durable, highly flexible, has low longitudinal shrinkage, and is available at a high Shore hardness of 72D.

By providing a cross-linked PEBA material in which the polyamide groups are substantially intact and free of cross-link, these groups remain available for further reaction, such as hydrogen bonding to other active groups, in this case, the amide groups of the adjacent nylon intermediate layer. Cross-linking PEBA in this manner provides the beneficial thermoset-like properties described above, but advantageously retains a modicum of desirable thermoplastic qualities, such as strength and elasticity. It also advantageously maintains a sufficient number of the polyamide functional groups in a condition suitable for later bonding to the nylon intermediate layer.

In most cases, it is expected that a highly cross-linked copolymer will be desirable for use as an outer jacket in an introducer sheath. A highly cross-linked outer layer has a high hoop strength (dimensional stability), a high hardness level, and an enhanced ability to shrink upon exposure to heat. As a result, the cross-linked heat shrink layer is very effective in compressing the intermediate layer onto the reinforcing layer and the inner liner. The degree of cross-linking may optimally be established by routine experimentation such that it is not so high that it results in a brittle outer layer, nor so low that it does not achieve the desired thermoset-like properties. Those skilled in the art will appreciate that the degree of cross-linking can be varied as desired, to achieve these desired properties, or others, for a particular sheath.

Although the outer layer has been described herein as comprising polyether block amide (PEBA), other cross-linkable polymers may similarly be used in a particular application. For best results, a copolymer formed of alternating hard blocks and amorphous blocks will be utilized. Similarly, the copolymer should be selected such that following cross-linking, the copolymer has a sufficient number of non-cross-linked active sites (analogous to the amide sites on the PEBA copolymer) that are capable of forming a secure bond with the adjoining inner layer of the sheath. A non-limiting list of other polymeric compositions that may be utilized as an outer heat shrink layer under appropriate conditions includes polyolefins, PET and FEP.

Similarly, although the introducer sheath was described as having an intermediate nylon layer, the intermediate layer may alternatively be formed of any material capable of forming a secure bond with the cross-linked copolymer of the outer layer. Those skilled in the art will appreciate that although a nylon intermediate layer and a PEBA outer layer have been described in the example provided herein, any other suitable combination of polymers may be utilized as an intermediate layer and an outer layer, such that the benefits of the present invention are obtainable thereby. Cross-linked PEBA is believed to be sufficiently bondable to a large number of polymers presently used in sheaths, and which polymers would be suitable for use herein. Non-limiting examples of such polymers include nylons and other polyamides, polyurethanes, certain grades of PEBA and polyethylene terephthalate (PET).

Although the sheath has been described herein as including a discrete inner liner, the sheath need not necessarily have a liner. Although such liners are often beneficial for a particular use of the sheath, this will not always be the case. In some instances, the layer in which the reinforcement is disposed may contain sufficient lubricity and hoop strength for the intended purpose of the sheath that the inner liner may be omitted. In other instances, the expected use of the sheath does not mandate the specific properties provided by the inner liner. When the inner liner is omitted, the layer referred to hereinabove as the "intermediate" layer will more properly be referred to as the inner layer, and the copolymer will continue to be referred to as the "outer" layer.

A method for forming a sheath according to the present invention will now be described. Conventional introducer sheaths typically comprise an inner fluorocarbon liner such as PTFE, a reinforcement layer comprising a braid and/or a coil, and an outer jacket formed of a heat-formable thermoplastic material such as nylon. In the prior art introducer sheaths, the thermoplastic heat formable material is heat shrunk onto the PTFE outer surface by enveloping the assembly in a heat shrink tube comprising FEP, PET or an olefm, and heating the entire assembly until the thermoplastic material melts. As this material melts, the shrinkage of the heat shrink material compresses the melted thermoplastic material and forces it to flow between the uniform spacings of the coils, thereby bonding the outer thermoplastic material to the outer diameter of the inner liner. The heat shrink tube does not bond to the nylon layer during heating. After the assembly has cooled, the heat shrink tube is skived from the assembly and discarded. A typical process for preparing such prior art introducers is described in U.S. Patent No. 5,380,304.

Preparation of the introducer sheaths of the present invention may be commenced in a manner similar to the preparation of the sheaths of the '304 patent. However, instead of enveloping the assembly (i.e., the inner liner, reinforcement and nylon layer) in a non-bonding heat shrink material as in the conventional process, the assembly in the inventive method is placed within a heat shrink material that is capable of bonding with the thermoplastic nylon layer via secondary molecular forces. Ideally, the intermediate layer will melt flow somewhere between the shrink temperature and the degradation temperature of the outer cross-linked layer. However, the intermediate layer may melt flow but not degradate at a temperature that is lower than the shrink temperature of the outer layer. Typically, the outer layer comprises a cross-linked block copolymer. Most preferably, the outer layer comprises cross-linked PEBA, as described herein.

The particular hoop strength of the resulting introducer sheath depends on several variables, such as the wall thickness and the Shore hardness of the sheath, as well as the degree of cross-linking of the material. Hoop strength may also depend on the diameter at which the shrink tube has recovered. Hoop strength tends to be weaker at maximum expansion and at the recovered diameter. It is typically highest at about half way to recovery. Those skilled in the art will appreciate that careful selection and control of these variables allows one to prepare a sheath having a desired hoop strength.

Because the PEBA heat shrink material bonds to the thermoplastic intermediate layer as described, care should be taken to ensure that air pockets are not trapped in the system when fusing. Trapped air pockets may form cavities, which have an adverse effect on the final product. There are two particularly preferred methods to fuse the outer layer to the intermediate layer in a manner to minimize the formation of air pockets. One such method involves fusing or bonding the respective layers in a longitudinal manner from one axial end (designated here as the first end) to the other axial end (designated here as the second end). This allows air to escape out the second end as the fusing progresses from the first end to the second end. This can be accomplished, e.g., using a heating element such as a die or a forced convection heater. The catheter assembly may also be passed through an RF coil that heats up the braid, coil or a fusing mandrel. In this instance, only the portion of the catheter that is directly within the RF coil will heat up, this allowing the operator to sequentially heat the catheter from one end to the other. A second method is to pull a vacuum, thereby evacuating any air in the catheter assembly. This would allow the catheter assembly to be fused in a regular convection oven.

Shrink temperatures for a sheath of the present invention can vary widely, depending on material. Polyolefin has a low shrink temperature of about 143°C (290°F), however it can withstand temperatures up to about 232°C (450°F). PET shrinks at 150°C (302°F) but melts at 190°C (374°F). This limited range limits the number of materials that it can fuse. PEBA shrinks at 171°C (340°F) and will not degrade at temperatures under about 260°C (500°F). FEP shrinks at 190°C (375°F), but does not degrade until temperatures exceed 315°C (600°F). The temperature range for PEB A gives it an advantage over PET because some thermoplastics require higher temperatures to melt. Polyolefin has a good temperature range, but does not have the same hoop strength as PEBA. FEP requires high temperatures, but generally must be provided with a thick wall (greater than about 0.13 mm). In view of these properties, and particularly its ability to laminate to the nylon intermediate layer in the preferred embodiment described herein, cross-linked PEBA is the preferred heat shrink material. A bond formed under these conditions typically has sufficient strength to resist delamination under conditions expected to be encountered during use of the sheath.

Unlike the prior art process, when the process of the present invention is utilized to form an introducer sheath it is not necessary to skive off and discard the material of the heat shrink envelope. Rather, the heat shrink material has sufficient hoop strength such that it is securely fused with the intermediate layer 35 (Fig. 2), 55 (Fig. 4), 75 (Fig. 5) under the conditions of the heat shrink operation. As a result, the material of the heat shrink envelope is simply left in place on the entire assembly after the assembly has cooled. Since the material need not be skived from the assembly, the efficiency of the operation, as well as its yield, are improved. Proper fusion of the heat shrink material onto the polymeric intermediate layer minimizes the risk of delamination of the heat shrink outer material during use of the sheath.

It is therefore intended that the foregoing detailed description be regarded as illustrative rather than limiting, and that it be understood that it is the following claims, including all equivalents, that are intended to define the scope of this invention.

## Claims

1. An introducer sheath comprising:
an inner liner having a passageway extending longitudinally therethrough;
a reinforcing member disposed radially outwardly from said layer;
an intermediate layer positioned longitudinally over said reinforcing member and inner liner, said intermediate layer connected to an outer circumferential surface of the inner liner between spacings of the reinforcing member, and wherein said intermediate layer is a thermoplastic; and
an outer layer positioned longitudinally over said intermediate layer, said outer layer comprising a polymer, wherein at least a portion of said polymer is at least partially cross-linked such that the polymer exhibits thermoset-like properties, and wherein said outer layer is bonded to an outer surface of said intermediate layer.

2. The introducer sheath of claim 1, wherein the material of said outer layer is a heat shrinkable material with a higher melting point than that of the material of said intermediate layer.

3. The introducer sheath of claims 1 or 2, wherein said outer layer comprises a block copolymer comprising alternate segments of hard and soft blocks, and said intermediate layer comprises a polymer having active functional groups; and wherein non-cross-linked segments of said block copolymer are bonded with said active functional groups of said polymeric intermediate layer.

4. The introducer sheath of claim 3, wherein said block copolymer comprises a polyether block amide having alternating hard polyamide blocks and soft polyether blocks, and said intermediate layer comprises nylon; and wherein non-cross-linked polyamide blocks are bonded to active amide groups of said nylon intermediate layer.

5. The introducer sheath of claim 4, wherein the polyether block amide includes urethane linking sites, and said polyether block amide is cross-linked at said urethane sites.

6. The introducer sheath of claim 4, wherein the polyether block amide has a Shore hardness between about 75 A and 72 D.

7. The introducer sheath of claim 1, wherein the reinforcing member comprises a braid, a coil, or a combination of a braid and a coil.

8. The introducer sheath of claim 1, wherein the inner layer comprises a discrete inner liner.

9. The introducer sheath of claim 8, wherein the inner liner comprises a fluorocarbon.

10. A method for forming an introducer sheath, comprising:
providing an assembly comprising an inner liner, a reinforcement disposed over said inner liner, and a polymeric intermediate layer disposed over said inner liner;
positioning the assembly within a heat shrinkable material, said heat shrinkable material comprising a polymeric material capable of bonding with the polymeric intermediate layer; and
heating the heat shrinkable material and the assembly positioned therein to a temperature sufficient to shrink the heat shrinkable material, and to melt the polymeric intermediate layer in a manner such that an outer surface of the melted polymeric intermediate material bonds to the heat shrinkable material and an inner surface of the melted polymeric intermediate layer bonds to the inner liner.

11. The method of claim 10, wherein said heat shrinkable material comprises a cross-linked copolymer having active functional groups, said active functional groups capable of bonding with active functional groups of said intermediate layer.

12. The method of claim 10, wherein said heat shrinkable material comprises a block copolymer comprising alternate segments of hard and soft blocks, and said intermediate layer has active functional groups; and wherein non-cross-linked segments of said block copolymer bond with said active functional groups of said intermediate layer.

13. The method of claim 12, wherein said block copolymer comprises a polyether block amide having alternating hard polyamide blocks and soft polyether blocks, and said intermediate layer comprises nylon, and wherein non-cross-linked polyamide blocks are bonded to active amide groups of said nylon intermediate layer.

14. The method of claim 13, wherein the polyether block amide includes urethane linking sites, and said polyether block amide is cross-linked at said urethane sites.

15. The method of claim 13, wherein said heating step is carried out at a temperature not exceeding about 260 °C.

16. The method of claim 11, wherein said reinforcement comprises a braid, a coil, or a combination of a braid and a coil, and wherein said reinforcement is at least partially embedded in said melted intermediate layer.

17. The method of claim 11, wherein the inner liner comprises PTFE, said reinforcement comprises a metal or a metal alloy, said polymeric intermediate layer comprises nylon, and said heat shrinkable material comprises polyether block amide.

18. The method of claim 10, wherein said polymeric intermediate layer is sequentially fused to the heat shrinkable material from one axial end of the heat shrink material to the other axial end.

19. The method of claim 10, wherein said heating step is carried out under a vacuum.

## Patentansprüche

1. Einführungshülse, umfassend:
eine Innenauskleidung mit einem Durchgang, welcher der Länge nach hindurch verläuft;
ein Verstärkungselement, das radial außerhalb dieser Schicht angeordnet ist;
eine Zwischenschicht, die der Länge nach über dem Verstärkungselement und der Innenauskleidung angeordnet ist, wobei die Zwischenschicht in Zwischenräumen des Verstärkungselements mit einer äußeren Umfangsoberfläche der Innenauskleidung verbunden ist, und wobei die Zwischenschicht ein Thermoplast ist; und
eine Außenschicht, die der Länge nach über der Zwischenschicht angeordnet ist, wobei die Außenschicht ein Polymer umfasst, wobei wenigstens ein Teil des Polymers wenigstens teilvernetzt ist, sodass das Polymer Duroplast-artige Eigenschaften aufweist, und wobei die Außenschicht an eine Außenoberfläche der Zwischenschicht gebunden ist.

2. Einführungshülse gemäß Anspruch 1, wobei das Material der Außenschicht ein wärmeschrumpfbares Material mit einem höheren Schmelzpunkt als der des Materials der Zwischenschicht ist.

3. Einführungshülse gemäß Anspruch 1 oder 2, wobei die Außenschicht ein Blockcopolymer umfasst, umfassend abwechselnde Segmente von harten und weichen Blöcken, und wobei die Zwischenschicht ein Polymer mit aktiven funktionellen Gruppen umfasst; und wobei nichtvernetzte Segmente des Blockcopolymers an die aktiven funktionellen Gruppen der polymeren Zwischenschicht gebunden sind.

4. Einführungshülse gemäß Anspruch 3, wobei das Blockcopolymer ein Polyetherblockamid mit abwechselnden harten Polyamidblöcken und weichen Polyetherblöcken umfasst und die Zwischenschicht Nylon umfasst; und wobei nichtvernetzte Polyamidblöcke an aktive Amidgruppen der Nylon-Zwischenschicht gebunden sind.

5. Einführungshülse gemäß Anspruch 4, wobei das Polyetherblockamid Urethan-Verknüpfungsstellen umfasst und das Polyetherblockamid an den Urethanstellen vernetzt ist.

6. Einführungshülse gemäß Anspruch 4, wobei das Polyetherblockamid eine Shore-Härte zwischen etwa 75 A und 72 D aufweist.

7. Einführungshülse gemäß Anspruch 1, wobei das Verstärkungselement ein Geflecht, eine Spirale oder eine Kombination eines Geflechts und einer Spirale umfasst.

8. Einführungshülse gemäß Anspruch 1, wobei die Innenschicht eine diskrete Innenauskleidung umfasst.

9. Einführungshülse gemäß Anspruch 8, wobei die Innenauskleidung einen Fluorkohlenstoff umfasst.

10. Verfahren zum Herstellen einer Einführungshülse, umfassend:
Bereitstellen einer Anordnung, umfassend eine Innenauskleidung, eine Verstärkung, die über der Innenauskleidung angeordnet ist, und eine polymere Zwischenschicht, die über der Innenauskleidung angeordnet ist;
Anordnen der Anordnung innerhalb eines wärmeschrumpfbaren Materials, wobei das wärmeschrumpfbare Material ein Polymermaterial umfasst, das mit der polymeren Zwischenschicht binden kann; und
Erwärmen des wärmeschrumpfbaren Materials und der darin angeordneten Anordnung auf eine Temperatur, die ausreicht, um das wärmeschrumpfbare Material zu schrumpfen und die polymere Zwischenschicht auf eine solche Weise zu schmelzen, dass eine äußere Oberfläche des geschmolzenen polymeren Zwischenmaterials an das wärmeschrumpfbare Material bindet und eine innere Oberfläche der geschmolzenen polymeren Zwischenschicht an die Innenauskleidung bindet.

11. Verfahren gemäß Anspruch 10, wobei das wärmeschrumpfbare Material ein vernetztes Copolymer mit aktiven funktionellen Gruppen umfasst, wobei die aktiven funktionellen Gruppen mit aktiven funktionellen Gruppen der Zwischenschicht binden können.

12. Verfahren gemäß Anspruch 10, wobei das wärmeschrumpfbare Material ein Blockcopolymer umfasst, das abwechselnde Segmente von harten und weichen Blöcken umfasst, und die Zwischenschicht aktive funktionelle Gruppen aufweist; und wobei nichtvernetzte Segmente des Blockcopolymers mit den aktiven funktionellen Gruppen der Zwischenschicht binden.

13. Verfahren gemäß Anspruch 12, wobei das Blockcopolymer ein Polyetherblockamid mit abwechselnden harten Polyamidblöcken und weichen Polyetherblöcken umfasst und die Zwischenschicht Nylon umfasst, und wobei nichtvernetzte Polyamidblöcke an aktive Amidgruppen der Nylon-Zwischenschicht gebunden sind.

14. Verfahren gemäß Anspruch 13, wobei das Polyetherblockamid Urethan-Verknüpfungsstellen umfasst und das Polyetherblockamid an den Urethanstellen vernetzt ist.

15. Verfahren gemäß Anspruch 13, wobei der Schritt des Wärmens bei einer Temperatur, die etwa 260 °C nicht übersteigt, durchgeführt wird.

16. Verfahren gemäß Anspruch 11, wobei die Verstärkung ein Geflecht, eine Spirale oder eine Kombination eines Geflechts und einer Spirale umfasst und wobei die Verstärkung wenigstens teilweise in der geschmolzenen Zwischenschicht eingebettet ist.

17. Verfahren gemäß Anspruch 11, wobei die Innenauskleidung PTFE umfasst, die Verstärkung ein Metall oder eine Metalllegierung umfasst, die polymere Zwischenschicht Nylon umfasst und das wärmeschrumpfbare Material Polyetherblockamid umfasst.

18. Verfahren gemäß Anspruch 10, wobei die polymere Zwischenschicht sequenziell von einem axialen Ende des wärmeschrumpfbaren Materials zum anderen axialen Ende an das wärmeschrumpfbare Material geschmolzen wird.

19. Verfahren gemäß Anspruch 10, wobei der Schritt des Wärmens unter einem Vakuum durchgeführt wird.

## Revendications

1. Gaine d'introduction comprenant :
une doublure interne comportant un passage qui s'étend longitudinalement à travers celle-ci ;
un élément de renforcement disposé radialement vers l'extérieur de ladite couche ;
une couche intermédiaire positionnée longitudinalement par-dessus ledit élément de renforcement et ladite doublure interne, ladite couche intermédiaire étant connectée à une surface circonférentielle externe de la doublure interne entre les espacements de l'élément de renforcement, et ladite couche intermédiaire étant un thermoplastique ; et
une couche externe positionnée longitudinalement par-dessus ladite couche intermédiaire, ladite couche externe comprenant un polymère, au moins une portion dudit polymère étant au moins partiellement réticulée de sorte que le polymère présente des propriétés semblables à celles d'un matériau thermodurci, et la couche externe étant liée à une surface externe de ladite couche intermédiaire.

2. Gaine d'introduction selon la revendication 1, dans laquelle le matériau de ladite couche externe est un matériau thermorétractable ayant un point de fusion supérieur à celui du matériau de ladite couche intermédiaire.

3. Gaine d'introduction selon la revendication 1 ou 2, dans laquelle ladite couche externe comprend un copolymère à blocs comprenant des segments alternés de blocs durs et mous, et ladite couche intermédiaire comprend un polymère comportant des groupes fonctionnels actifs ; et dans laquelle des segments non réticulés dudit copolymère à blocs sont liés auxdits groupes fonctionnels actifs de ladite couche intermédiaire polymère.

4. Gaine d'introduction selon la revendication 3, dans laquelle ledit copolymère à blocs comprend un amide à blocs polyéthers comportant des blocs polyamides durs et des blocs polyéthers mous alternés, et ladite couche intermédiaire comprend du nylon ; et dans laquelle des blocs polyamides non réticulés sont liés à des groupes amides actifs de ladite couche intermédiaire comprenant du nylon.

5. Gaine d'introduction selon la revendication 4, dans laquelle l'amide à blocs polyéthers contient des sites de liaison uréthanes, et ledit amide à blocs polyéthers est réticulé au niveau desdits sites uréthanes.

6. Gaine d'introduction selon la revendication 4, dans laquelle l'amide à blocs polyéthers a une dureté Shore d'environ 75 A à 72 D.

7. Gaine d'introduction selon la revendication 1, dans laquelle l'élément de renforcement comprend une tresse, une spirale, ou une combinaison d'une tresse et d'une spirale.

8. Gaine d'introduction selon la revendication 1, dans laquelle la couche interne comprend une doublure interne discontinue.

9. Gaine d'introduction selon la revendication 8, dans laquelle la doublure interne comprend un fluorocarbure.

10. Procédé de fabrication d'une gaine d'introduction, comprenant :
la fourniture d'un assemblage comprenant une doublure interne, un renforcement disposé par-dessus ladite doublure interne, et une couche intermédiaire polymère disposée par-dessus ladite doublure interne ;
le positionnement de l'assemblage à l'intérieur d'un matériau thermorétractable, ledit matériau thermorétractable comprenant un matériau polymère capable de se lier à la couche intermédiaire polymère ; et
le chauffage du matériau thermorétractable et de l'assemblage positionné à l'intérieur de celui-ci à une température suffisante pour causer une rétraction du matériau thermorétractable, et pour faire fondre la couche intermédiaire polymère de manière telle qu'une surface externe du matériau intermédiaire polymère fondu se lie au matériau thermorétractable et qu'une surface interne de la couche intermédiaire polymère fondue se lie à la doublure interne.

11. Procédé selon la revendication 10, dans lequel ledit matériau thermorétractable comprend un copolymère réticulé comportant des groupes fonctionnels actifs, lesdits groupes fonctionnels actifs étant capables de se lier avec des groupes fonctionnels actifs de ladite couche intermédiaire.

12. Procédé selon la revendication 10, dans lequel ledit matériau thermorétractable comprend un copolymère à blocs comprenant des segments alternés de blocs durs et mous, et ladite couche intermédiaire comporte des groupes fonctionnels actifs ; et dans lequel des segments non réticulés dudit copolymère à blocs se lient avec lesdits groupes fonctionnels actifs de ladite couche intermédiaire.

13. Procédé selon la revendication 12, dans lequel ledit copolymère à blocs comprend un amide à blocs polyéthers comportant des blocs polyamides durs et des blocs polyéthers mous alternés, et ladite couche intermédiaire comprend du nylon ; et dans lequel des blocs polyamides non réticulés sont liés à des groupes amides actifs de ladite couche intermédiaire comprenant du nylon.

14. Procédé selon la revendication 13, dans lequel l'amide à blocs polyéthers contient des sites de liaison uréthanes, et ledit amide à blocs polyéthers est réticulé au niveau desdits sites uréthanes.

15. Procédé selon la revendication 13, dans lequel ladite étape de chauffage est exécutée à une température ne dépassant pas environ 260 °C.

16. Procédé selon la revendication 11, dans lequel ledit renforcement comprend une tresse, une spirale, ou une combinaison d'une tresse et d'une spirale, et dans lequel ledit renforcement est au moins en partie encastré dans ladite couche intermédiaire fondue.

17. Procédé selon la revendication 11, dans lequel la doublure interne comprend du PTFE, ledit renforcement comprend un métal ou un alliage de métal, ladite couche intermédiaire polymère comprend du nylon, et ledit matériau thermorétractable comprend un amide à blocs polyéthers.

18. Procédé selon la revendication 10, dans lequel ladite couche intermédiaire polymère est soudée séquentiellement au matériau thermorétractable d'une extrémité axiale du matériau thermorétractable à l'autre extrémité axiale.

19. Procédé selon la revendication 10, dans lequel ladite étape de chauffage est exécutée sous vide.
